Europäisches Patentamt

European Patent Office

Office européen des brevets

(11) Publication number: **0 236 330 B1**

# EUROPEAN PATENT SPECIFICATION

(45) Date of publication of patent specification: **23.06.93**   (51) Int. Cl.5: **C07K 15/00**, C12N 1/20, C12R 1/19

(21) Application number: **86900122.2**

(22) Date of filing: **12.12.85**

(86) International application number:
**PCT/EP85/00698**

(87) International publication number:
**WO 86/03517 (19.06.86 86/13)**

(54) **DNA SEOUENCE ENCODING A HIRUDIN-LIKE PROTEIN AND PROCESS FOR PREPARING SUCH PROTEIN.**

(30) Priority: **13.12.84 DE 3445517**

(43) Date of publication of application:
**16.09.87 Bulletin 87/38**

(45) Publication of the grant of the patent:
**23.06.93 Bulletin 93/25**

(84) Designated Contracting States:
**AT BE CH DE FR GB IT LI LU NL SE**

(56) References cited:
**EP-A- 0 171 024
EP-A-01 683 42
WO-A-85/04418**

**FEBS 1044, vol. 164, no.2, Jan. 1984, pages 307-313, J. Dodt et al.**

(73) Proprietor: **CIBA-GEIGY AG
Klybeckstrasse 141
CH-4002 Basel(CH)**

Proprietor: **UCP GEN-PHARMA AG
Solothurnstrasse 24
CH-3422 Kirchberg(CH)**

(72) Inventor: **FORTKAMP, Elke
Oberer Rainweg 29
W-6900 Heidelberg(DE)**
Inventor: **RIEGER, Michael
Angelhofweg 39
W-6916 Wilhelmsfeld(DE)**
Inventor: **SOMMER, Reinhold
Furtwänglerstr. 39
W-6900 Heidelberg(DE)**
Inventor: **FINK, Ernest
Stellhorner Strasse 1
W-2910 Westerstede-Giesselhorst(DE)**

Rank Xerox (UK) Business Services
(3.10/3.6/3.3.1)

Chemical Abstracts, vol. 100, no.19, 7 May 1984 (Columbus, Ohio, US), J. Dodt et al "The complete amino acid sequence of hirudin, a thrombin specific inhibitor. Application of color carboxymethylation", see page 227, abstract no.152937g

## Description

The invention relates to a DNA sequence encoding a hirudin-like protein having the biological activity of hirudin. The invention further relates to recombinant DNA molecules, i.e. cloning and expression vectors, for use in the preparation of a hirudin-like protein having the biological activity of hirudin and to host organisms, such as bacteria, yeasts and mammalian cells, transformed with such vectors. The invention finally relates to pharmaceutical compositions containing a hirudin-like protein having the biological activity of hirudin.

To apply leeches (Hirudo medicinalis) is one of the oldest known therapy forms. It can be proven that already in prehistoric times extractions of blood were made as therapeutic treatment.

For thousands of years the leech therapy has offered a therapeutic method which brought better results than the mere extraction of blood. However, nothing was known about its mode of action.

The first historic therapy using leeches was employed by the Greek physician Nikander of Colophon almost 2200 years ago; however, Themison of Laodica, a disciple of Aesculap, is regarded to be the actual founder of this therapy form. In the Middle Ages the leeches were generally used for healing purposes, not only by the barbers, the male nurses of that time, but also in particular by the physicians.

A leech can take up about 15 cubic centimeters of blood within a few minutes. It would be choked by this food if it could not prevent the blood from coagulating. Already in the wound the leech admixes an anticoagulant to the blood. The secretion of a thrombin antagonist is absolutely vital for the leech. The leech produces the thrombin antagonist in its jugular glands.

The anticoagulant property additionally improves blood circulation, increases the blood and lymph stream rate by several times and has a vasospasmolytic effect. Moreover, this secretion has antibiotic properties which only the bacterium Pseudomonas survives. This bacterium digests the stored blood and transforms it into nourishment. If this bacterium is destroyed, the leech dies, since without it the leech is not viable. Thus it becomes understandable why the leech has been a helpful companion of mankind for thousands of years. Its secretion is antithrombotic, anti-inflammatory, edema-reductive, accelerates the rate of the lymph and blood streams, is vasospasmolytic and antibiotic. Thus it is a highly effective substance. Small amounts in a bite caused by a leech (the bite, by the way, does not hurt, but only causes slight itching) prevent the coagulation of blood for up to 10 hours.

After asepsis and antisepsis ended the direct therapy with leeches, researchers all over the world looked for a way to isolate the active substance in order to use it again in therapy. In 1884 for the first time an anticoagulant extract was obtained from leeches. Later the active substance of the leech was partly purified from foreign protein. The obtained crude material was designated hirudin.

Hirudin is a high molecular polypeptide having a molecular weight of 7000 and anti-inflammatory and edema-reductive properties. The analysis of the inhibitory effect on the individual phases of the coagulation system showed that it was a specific inhibitory substance of the enzyme thrombin which does not react with any other coagulant factor and produces its effect without the cooperation of other components present in the blood. Hirudin thus prevents the coagulation of blood by intercepting the reaction product of the first coagulation phase, namely the thrombin, and thus prevents the triggering of the second coagulation phase, namely the formation of fibrin.

Hirudin is therefore a specific thrombin antagonist (Jui-Yoa Chang, FEBS Lett. Vol 164, page 307 (1983)). So far, hirudin has been isolated from leeches. Only about 1 mg hirudin could be obtained from 70 leeches. The hirudin thus obtained was mainly processed to ointments used for the antithrombotic and antiphlogistic vein therapy (P. Walsmann, F. Markwardt, Die Pharmazie, Vol. 36, page 653 (1981)). Of late, hirudin has also been used in the treatment of clotting disorders or for the prevention of blood coagulation, e.g. during hemodialysis. However, because it was only available in limited amounts and since its purity was not sufficient, hirudin hitherto could not be used in clinical tests.

The not prepublished EP-A-158564 and 168342 disclose DNA sequences encoding hirudin and a process for producing this protein. The complete amino acid sequence of hirudin was described by J. Dodt et al in FEBS Letters, Vol. 165, No.2 pp. 180-184.

The problem underlying the present invention is to provide a DNA sequence which encodes a hirudin-like protein having the biological activity of hirudin and a process which enables large scale production of this protein by genetic engineering.

The invention thus relates to the subject matter characterized in the patent claims.

The term "hirudin-like protein" designates a protein whose amino acid sequence is similar to the amino acid sequence of the natural hirudin or only comprises part thereof and/or is not sulfatized at the tyrosin residue 63 in contrast to the natural hirudin. According to the present invention, corresponding fusion proteins are also designated as hirudin-like proteins. The designation "protein having the biological activity of hirudin" relates to a protein which does not need to be identical with the natural hirudin but which nevertheless has the biological activity of natural hirudin. Thus it acts vis-à-vis proteins, such as thrombin, like the natural hirudin and/or has immunological properties of the natural hirudin.

It is known that the desulfatization of hirudin causes only a slight reduction in the biological activity of hirudin (Jui-Yoa Chang, FEBS Lett., Vol. 164, page 307 (1983)).

For the preparation of a hirudin-like protein having the biological activity of hirudin by genetic engineering the following steps are carried out:

First the oligodeoxyribonucleotides listed in table I are synthesized using a DNA synthesizer. This synthesis is based on the amino acid sequence described by J. Dodt, H.P. Müller, U. Seemüller and Jui-Yoa Chang in FEBS Lett., Vol. 615, page 180 (1984). Predominantly employed are the codons most frequently used by E. coli.

Care is taken that at the 5' end of the synthetic gene there is an ATG codon, because in this case optionally the corresponding protein can be cleaved from a fusion protein by CNBr cleavage. The oligodeoxyribonucleotides thus obtained are purified and phosphorylated with polynucleotidekinase and $\gamma$-$^{32}$P-rATP because of the ligation to be carried out at a later stage.

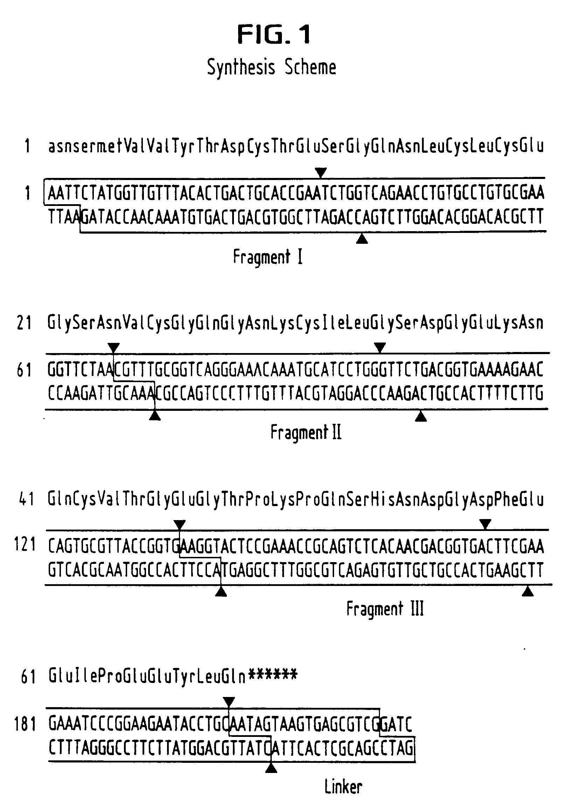

The products of this reaction are purified by a preparative polyacrylamide-gel electrophoresis. Subsequently the respective + and - strands (complementary strands) are annealed, ligated to yield gene fragments, which are then purified by a preparative polyacrylamide-gel electrophoresis and joined together to the complete synthetic gene (Fig. 1). The gene thus obtained can be used for isolating the natural gene from genomic libraries or from cDNA libraries.

This synthetic gene is inserted into the vector pEMBL 8+ (deposited with DSM, Göttingen, Federal Republic of Germany under the deposition number DSM 3139) which contains the promoter lac UV5. Of course, other expression control sequences may be used as well, e.g. the E. coli tac system, the E. coli trp system, the E. coli lipoprotein promoter, yeast expression control sequences or other eukaryotic expression control sequences.

What is important in this context is the operative linkage of the gene with the expression control sequence as well as the selection of a suitable expression control sequence for a particular host organism.

After the recombinant expression vector has been constructed it is inserted into a transformable host organism, such as a coli bacterium, e.g. E. coli OM 214 (Stammsammlung Prof. G. Schütz, Deutsches Krebsforschungszentrum Heidelberg; DSM Göttingen, Deposition Number DSM 3138). Depending on the expression control sequence, other bacteria, yeasts, and animal or human cells may also be used as host organism. Transformants synthesizing a hirudin-like protein are identified by their ampicillin resistance and by reaction with anti-hirudin-IgG antibodies.

Finally, the hirudin-like protein prepared according to the invention is tested for its biological activity vis-à-vis the enzyme thrombin which plays a key role in blood coagulation.

In this connection it was found that firstly the hirudin-like protein produced from bacteria inhibits the reaction from fibrinogen to fibrin by thrombin and secondly that with this protein also the transformation of a chromogenic substance by thrombin may be inhibited. From this can be seen that the protein produced according to the process of the present invention has the biological activity of hirudin.

Thus, pharmaceutical compositions for the treatment of coagulation disorders of the blood or for the prevention of blood coagulation may be produced using the inventive protein and its cyanogen bromide cleavage product, respectively. Optionally, pharmaceutically acceptable additives and diluents can be added.

Fig. 1: shows the complete DNA sequence of the synthetic gene. The black arrows designate the boundaries of the synthesized single-stranded oligodeoxynucleotides, the subfragments I, II and III as well as the linker fragment are boxed. Moreover, the corresponding amino acid sequence is shown above the DNA sequence.

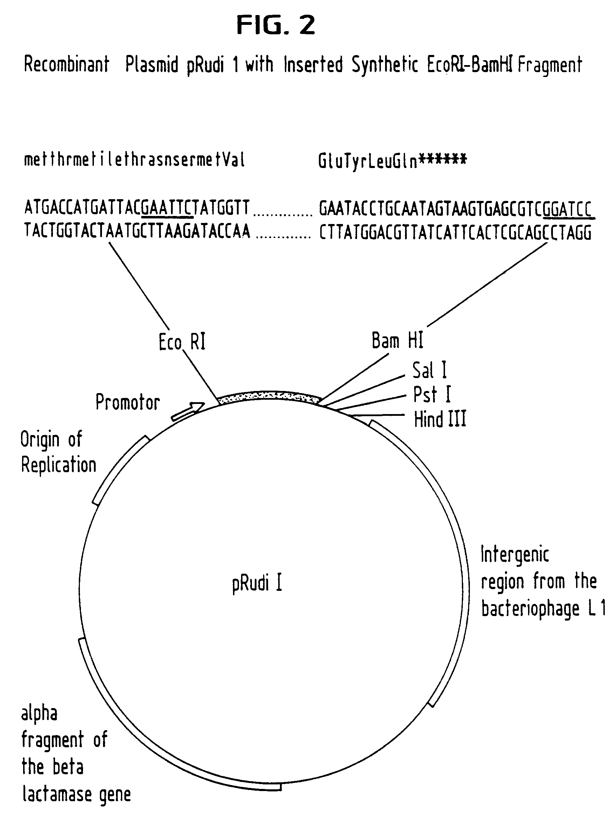

Fig. 2: shows the recombinant plasmid pRudi 1 with an inserted synthetic EcoRI-BamHI fragment. The synthetic fragment was inserted into the poly-linker located in the alpha fragment of the $\beta$-galactosidase gene of plasmid pEMBL 8+. The first amino acids of the alpha fragment form the described fusion protein together with the amino acids of hirudin.

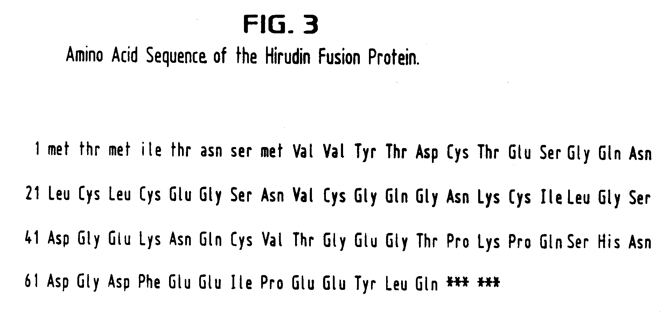

Fig. 3: shows the complete amino acid sequence of the fusion protein encoded by clone pRudi 1. The amino acids corresponding to natural hirudin are given starting with capital letters.

The following examples illustrate the present invention. They are given for illustrative purposes only and are by no means intended to limit the scope of the present invention.

Example 1

Synthesis of a DNA sequence encoding a protein having the biological activity of hirudin

The oligodeoxyribonucleotides listed in Table 1 are synthesized using a DNA synthesizer (Applied Biosystems Model 380A).

## Table I:  Synthesized Oligonucleotides

| | 5' End | 3' End |
|---|---|---|
| R1 | AATTCTATGGTTGTTTACACTGACTGCACCGAA | |
| R2 | CCAGATTCGGTGCAGTCAGTGTAAACAACCATAG | |
| R3 | TCTGGTCAGAACCTGTGCCTGTGCGAAGGTTCTAA | |
| R4 | AAACGTTAGAACCTTCGCACAGGCACAGGTTCTGA | |
| R5 | CGTTTGCGGTCAGGGAAACAAATGCATCCTGG | |
| R6 | AGAACCCAGGATGCATTTGTTTCCCTGACCGC | |
| R7 | GTTCTGACGGTGAAAAGAACCAGTGCGTTACCGGTG | |
| R8 | ACCTTCACCGGTAACGCACTGGTTCTTTTCACCGTC | |
| R9 | AAGGTACTCCGAAACCGCAGTCTCACAACGACGGTGA | |
| R10 | CGAAGTCACCGTCGTTGTGAGACTGCGGTTTCGGAGT | |
| R11 | CTTCGAAGAAATCCCGGAAGAATACCTGC | |
| R12 | CTATTGCAGGTATTCTTCCGGGATTTCTT | |
| R13 | AATAGTAAGTGAGCGTCG | |
| R14 | GATCCGACGCTCACTTA | |

The synthesis program is ABI 003 5/17/84. The chemicals used are also from Applied Biosystems, as is the LCAA/CPG (long chain alkylamine controlled pore glass) which has been functionalized with nucleoside-3-O-succinate (about 30 $\mu$mol/g). Each synthesis is started with 1 $\mu$mol nucleoside. The synthesis is terminated in each case with the "trityl off, auto" cleavage modus, with which the acid-instable 5'-O-(4,4'-dimethoxytrityl) group is removed before treatment with thiophenol and ammonia. The ammoniacal solutions of the products of the synthesizer are transferred into tapered flasks and the original glass containers are rinsed out with about 10 ml 25% ammonia solution each.

The flasks are carefully sealed and then heated at 50° C for 14 hours. Subsequently the solution is concentrated to dryness in the oil pump vacuum and each product is dissolved in 500 $\mu$l 20 mM triethylammonium bicarbonate buffer (TEAB), pH 7.4.

The tenth part of each crude oligomer is then purified by ion exchange HPLC (high performance liquid chromatography). The Latek HPLC system comprises two P 400 solvent pumps, an automatic gradient control, a Rheodyne 7125 injection member with a 100 $\mu$l sample reception loop, a Milton Roy Model 1204 A UV detector (detection wave length 280 nm, sensitivity 1.0) and a Shimadzu C-R3A recorder. The Latek Partisil 10 SAX HPLC column (4 mm in diameter) is located in a water bath with a temperature of 50° C, and the flow velocity is 2 ml/min. For separation,

a linear gradient of the solvents A and B of from 20 to 60% B is used for 45 minutes (solvent A = 40 % (v/v) 1 mM triethylammonium phosphate, pH 6.3, 60 % (v/v) formamide; solvent B = 40 % (v/v) 500 mM triethylammonium phosphate, pH 6.3, 60 % (v/v) formamide). In order to prepare these solutions analytical grade formamide is used which has been stirred for 5 hours with 40 g/l Bio Rad AG 501-X8 ion exchanger (analytical grade mixed bed resin).

The elution is carefully watched and the fraction with the last eluted UV-active substance (usually the main absorption) is manually collected. For desalting, distilled water is added to this fraction in a ratio of 1 : 1. The fraction is then applied to a 3 ml octadecyl ($C_{18}$) one way column (J.T. Baker 7020-3) which was pre-washed with 3 ml methanol, 200 mM TEAB, pH 7.4/30 % acetonitrile and 200 mM TEAB, pH 7.4, respectively. Afterwards the column is rinsed twice with 3 ml each of the same buffer. The product is subsequently subjected to elution with 2 x 3 ml 200 mM TEAB, pH 7.4/80 % methanol (H. Blöcker, R. Frank and assistants, EMBO Course, Braunschweig, September 1984, Manual). After concentration to dryness in the oil pump vacuum, the purified oligodeoxyribonucleotide is taken up in 1 ml 20 mM TEAB, pH 7.4, and the absorption is determined at 260 nm.

500 picomoles of each oligomer are employed for quantitative phosphorylation. The molar extinction coefficients are calculated by adding the individual extinction coefficients of the nucleotides without taking into consideration the hypochromatic effects. 500 picomoles of each oligonucleotide were suspended in 15 $\mu$l of distilled water and 2 $\mu$l of 10 x PNK buffer (500 mM Tris-HCl, pH 7.6, 100 mM MgCl$_2$, 50 mM DTT, 10 mM spermidine) and then kept at 70° C for 10 minutes. The mixture was subsequently chilled and adjusted to 1 $\mu$M of rATP, then 1 $\mu$Ci of adenosine 5'-($\gamma$-$^{32}$P) triphospate, triethylammonium salt (6000 Ci/mMol, Amersham Buchler GmbH & Co. KG, Braunschweig), and 1 $\mu$l of polynucleotidekinase (4.5 U, Boehringer Mannheim) were added. The reaction was incubated at 37° C. After 15 minutes 1 $\mu$l of 10 mM rATP was added and the incubation was continued for additional 15 minutes and stopped by the addition of 10 $\mu$l Urea-Mix (10 M urea, 20 mM EDTA, 0.05 % bromophenolblue, 0.05 % Xylenecyanolblue). Prior to gel loading, the mixture was kept at 95° C for 1 minute.

A denaturing polyacrylamide gel (60 cm long, 0.1 cm thick; 8 % acrylamide, 8.5 M urea, 1 x TBE (89 mM Tris base, 89 mM boric acid, 2 mM EDTA)) was prerun at 30 watt for 2 hours. The denatured phosporylation reactions were then loaded. The electrophoresis was performed at 30 watt until the bromophenolblue had migrated 45 cm. One of the glass plates was lifted off, the gel was covered with Saran wrap and exposed to an X-ray film for 1 hour.

The bands corresponding to the oligonucleotides of total length were cut out including the gel matrix. The matrix was crushed and suspended in 300 $\mu$l TE-buffer (10 mM Tris-HCl, pH 8, 1 mM EDTA).

The oligonucleotides were eluted at 65° C for 3 hours.

The gel matrix was then centrifuged down at 12,000 rpm for 10 minutes and the supernatant was ethanol-precipitated in the presence of 0.3 M sodium acetate, pH 10, and glycogen as a carrier. The pellet was washed two times with 80 % ethanol and dried under vacuum and taken up in TE-buffer to a final concentration of 2 picomoles of nucleotide/$\mu$l.

In a first step 50 picomoles of single-stranded oligonucleotide were annealed to its corresponding opposite strand in a total reaction volume of 100 $\mu$l (50 mM NaCl, 2 x TE, pH8).

The mixtures were heated up to 90° C, kept at this temperature for 2 minutes in a water bath which was then allowed to cool down to room temperature overnight.

Subfragments were ligated using 20 $\mu$l of each annealing mixture (subfragment I: R1 + R2 plus R3 + R4, subfragment II: R5 + R6 plus R7 + R8, subfragment III: R9 + R10 plus R11 + R12, subfragment IV: R13 + R14 were ligated to form a dimer) in a total volume of 100 $\mu$l (50 mM Tris-HCl, pH 7.5, 10 mM MgCl$_2$, 5 $\mu$g BSA, 1mM spermidine, 1mM ATP, 10 U of T4-DNA-ligase (Boehringer Mannheim)).

Incubation was carried out at 17° C for 4 hours and stopped by heating at 70° C for 10 minutes. The mixture was ethanol-precipitated in the presence of 0.3 M sodium acetate, pH 10, and glycogen as a carrier, washed twice with 80 % ethanol, dried and taken up in 30 $\mu$l TE-buffer, pH8.

20 $\mu$l of each ligation mixture were mixed with 5.5 $\mu$l loading buffer (20 % Ficoll 400 (Pharmacia, Uppsala, Sweden), 2 % SDS, 0.05 % bromophenolblue, 0.05 % xylene xyanol blue) and heated at 70° C for 5 minutes.

A non-denaturing polyacrylamide gel (40 cm long, 0.01 cm thick, 10 % acrylamide, 1 x TBE) was prerun at 200 volts for 2 hours, then the ligation samples were loaded. Electrophoresis was carried out at 300 volts in 1/2 x TBE for 7 hours until bromophenolblue migrated 25 cm. The gel was autoradiographed for 12 hours.

The fragments corresponding to the expected length were cut out and eluted as described for the first gel. The dried DNA pellet was taken up in 20 $\mu$l TE-buffer.

Approximately 1 picomole (10 $\mu$l of subfragment preparation) of each subfragment was ligated in a total reaction volume of 50 $\mu$l under the same conditions as described before. Inactivation of the enzyme and concentration of the DNA were carried out as already described. The DNA pellet was taken up in 8 $\mu$l TE. The total sequence of the synthetic gene is also shown in Figure 1.

Example 2

Construction of a Vector for Cloning, Expression of the Synthesized DNA Sequence and Transformation of E. coli OM 214

As the synthesized gene may form multimers via its EcoRI and BamHI cloning sites the preparation was digested with EcoRI and BamHI prior to ligation with the vector. 5 $\mu$l of the ligation reaction were digested with 40 units of EcoRI and 30 units of BamHI in a total volume of 40 $\mu$l (50 mM Tris, pH 7.5, 100 mM NaCl, 10 mM $MgCl_2$, 1 mM DTT) at 37° C for one hour. Thereafter the DNA was ethanol-precipitated in the presence of 0.3 M sodium acetate, pH 10, washed twice with 80% ethanol, dried and taken up in 24 $\mu$l TE.

10 $\mu$g plasmid DNA of pEMBL8 + (Dente, L., Cesareni, G. and Cortese, R. (1983), Nucleic Acids Res. 11, 1645-1655) were digested with 40 units EcoRI, 30 units BamHI and dephosphorylated with 2.5 units calf intestinal phosphatase (Boehringer Mannheim) in a total reaction volume of 100 $\mu$l at 37° C for 1 hour. The reaction buffer was 50 mM Tris, pH 7.5; 100 mM NaCl; 10 mM $MgCl_2$; 1 mM DTT. The reaction was stopped by the addition of EGTA to a final concentration of 25 mM and heating at 70° C for 10 minutes and then extracted with 150 $\mu$l of 80 % buffered phenol, pH 7.5.

The upper phase was applied to a gelfiltration column, filled with Sephadex G 100 (Pharmacia, Uppsala, Sweden; total bed volume: 2 ml), and equilibrated with 10 mM ammonium bicarbonate. Each fraction of the eluate contained approximately 100 $\mu$l. Aliquots of these fractions were spotted on agarose plates containing ethidium bromide. The DNA were visualized by UV illumination. The first fractions containing about 75 % of the whole DNA were pooled and dried under reduced pressure. The DNA was resuspended to a final concentration of 0.1 $\mu$g DNA/$\mu$l TE buffer.

The amount of 0.05 picomoles of the restricted insert DNA was mixed with 0.1 picomoles of prepared vector DNA in a total volume of 20 $\mu$l (50 mM Tris, pH 7.5; 10 mM $MgCl_2$; 1 $\mu$g BSA; 1 mM spermidine; 1 mM ATP; 5 units T4-DNA-ligase (Boehringer Mannheim)). The incubation was carried out at 14° C for 3 hours. After addition of 2 $\mu$g tRNA from calf thymus (Boehringer Mannheim) and 80 $\mu$l TE buffer the mixture was extracted with 100 $\mu$l of 80 % phenol, pH 7.5 - chloroform - isoamylalcohol (24 : 24 : 1; vol.:vol.:vol.). 10 $\mu$l of 3 M sodium acetate, pH 10, and 1 $\mu$l of glycogen (Renner GmbH, Darmstadt, West Germany) were added and the solution was ethanol-precipitated, washed twice with 80 % ethanol, dried under reduced pressure and redissolved in 10 $\mu$l TE buffer. The structure of the thus obtained plasmid pRudi 1 is shown in Figure 2. The amino acid sequence of the expression product is shown in Fig. 3. The expression product is a fusion protein with amino acids of the alpha peptide of the $\beta$-galactosidase (positions 1 - 8) and with the amino acids corresponding to the natural hirudin (positions 9 to end).

The preparation of competent E. coli strain OM 214 cells was done according to standard methods. 40 ml of nutrient broth (Serva, catalogue no. 48498) were inoculated with a single colony of E. coli strain OM 214. The culture was shaken at 200 rpm and 37° C for about 15 hours. 4 ml of this culture were added to 200 ml of fresh nutrient broth. Incubation was done as before until a cell density of 2 x $10^8$ cells per ml was reached. The culture was chilled quickly on ice and centrifuged at 8,000 rpm for 5 minutes. The sediment of the cells was resuspended in 100 ml 30 mM calcium chloride solution and kept at 0° C for 20 minutes, it was centrifuged again and resuspended in 10 ml calcium chloride solution. The cells were kept at 0° C for 24 hours. Then 1 ml of sterile glycerol was added and the cells were quickly frozen in liquid nitrogen and stored at - 70° C.

The thus obtained frozen competent E. coli cells were thawed on ice. To 100 $\mu$l of cells 5 $\mu$l of the purified ligated DNA were added. The mixture was kept at 0° C for 20 minutes. The cells were heat pulsed at 37° C for exactly 4 minutes to enable their uptake of plasmid DNA. The cell suspension was chilled on ice and diluted ten times into nutrient broth and incubated at 37° C for about 40 minutes. Aliquots (1%, 10 %, and 90 %) of the suspension were plated on agar plates containing 1.5 % agar, nutrient broth and 100 $\mu$g ampicillin per ml, which is used to select transformed cells by their resistance to that antibiotic. The agar plates were incubated at 37° C for 14 hours. The growing colonies were picked and checked for expression of a fusion protein with hirudin sequences by an enzyme-linked immunosorbent assay (ELISA).

Example 3

Expression of the Synthetic DNA Sequence and Detection of the Expression Products

2.5 ml 2 x YT medium containing 60 $\mu$g/ml ampicillin are incubated with individual E. coli colonies, and the cultures are shaken at 37° C for 6 hours. The cells are harvested by centrifugation, washed in 1 ml 10 mM NaCl and suspended in 250 $\mu$l Lysis buffer (50 mM Tris-HCl, pH8, 30 mM NaCl, 1 mg/ml Lysozym). After 30 minutes at 0° C the cells are frozen three times at -70° C and thawed at 37°C. The chilled lysates are centrifuged at 15,000 rpm. The supernatants are kept for detection of hirudin-like proteins.

The detection of hirudin in E. coli lysates is carried out with an ELISA test. 50 $\mu$l of an anti-hirudin-IgG solution (Plantorgan, Bad Zwischenahn) and 10 $\mu$l/ml IgG in linkage buffer (1.59 g $Na_2CO_3$, 2.93 g $NaHCO_3$, 0.2 g $NaN_3$, ad 1l $H_2O$) are filled into the wells of a microtiter plate and incubated overnight at 4° C. The wells are rinsed three times with PBS buffer (8.0 g NaCl, 0.2 g $KH_2PO_4$, 2.9 g $Na_2PO_4$.12 $H_2O$, 0.2 g KCl, 0.5 ml Tween 20, 0.2 g $NaN_3$, ad 1 l $H_2O$). This buffer is used for all rinsing procedures. 50 $\mu$l of the lysates to be tested are used. After 2 hours at room temperature the wells of the plates are washed three times and 50 $\mu$l anti-hirudin-IgG which was conjugated with alkaline phospatase are added. The conjugate is prepared with alkaline phosphatase type VII T (Sigma) according to the prescription given in the catalogue "Biochemical and Organic Compounds for Research", Sigma Chemie GmbH, page 713.

After incubation at room temperature for 2 hours the wells are again washed three times. Subsequently 50 $\mu$l of the substrate for alkaline phosphatase (Sigma 104: p-nitrophenylphospate, 1 mg/ml in 10 % diethanolamine, 0.1 mg/ml $MgCl_2$.6 $H_2O$, pH 9.8) are added. The enzyme reaction is visually determined. A positive result, i.e. the presence of hirudin-like protein can be seen from the yellow coloring of the solution due to the activity of the alkaline phosphatase linked to the anti-hirudin-IgG. A quantitative appraising of the hirudin-like protein in E. coli extracts is done by comparison with a dilution series of a hirudin standard solution of known concentration which is employed parallel to the test samples.

Thirty-five E. coli clones are tested altogether. Four of them show a positive result (Nos. 9, 13, 19, 35).

The comparison of the color reaction with that of the hirudin-standard solutions showed that the clones produced between 10 and 20 $\mu$g/l hirudin-like protein.

Example 4

Detection of the Biological Activity of the Hirudin-Like Protein

1 l 2 x YT medium containing 60 $\mu$g/ml ampicillin is incubated with 10 ml of an overnight culture of the E. coli clone No. 9 and shaken at 37° C. 5 ml 100 mM IPTG (Isopropyl-$\beta$-D-thio-galacto-pyranoside; Boehringer Mannheim) are added after 1 hour in order to induce the expression of the synthetic gene. After 5 more hours the cells are harvested, suspended in 100 ml Lysis buffer and lysated as described above. The lysate is brought to a final concentration of 10 mM with 1 M $MgCl_2$, and 10 mg DNase I are added. After 15 minutes at 37° C the lysate is incubated at 70° C for 15 minutes. Precipitated protein is centrifuged, the supernatant is titrated to a pH of 4.5 with trichloroacetic acid (6 %). Cold acetone is added to a final concentration of 50 %. After 2 hours at 0° C the precipitate is centrifuged. Cold acetone is added to the supernatant to a final concentration of 80 %. It is then kept at 0° C for 2 hours. The precipitate is then centrifuged again, the acetone is evaporated at 37° C and the precipitate is dissolved in 2 ml 50 mM Tris-HCl, pH 7.

Subsequently a clotting test is carried out with the E. coli extract. The clotting test is based on the specific inhibition of thrombin by hirudin or a protein having the biological activity of hirudin. A given amount of thrombin is added to a fibrinogen solution of known concentration. By this the transformation into fibrin is catalyzed and registered within a certain time. If hirudin or a protein having the biological activity of hirudin is added to the fibrinogen solution in increasing amounts, clotting is prevented as long as there is no excess thrombin.

The turning point can be recognized by the clotting of the sample.

The test is carried out as follows: 100 $\mu$l of a fibrinogen solution (60 mg/ml in 0.85 % NaCl) and 100 $\mu$l of the sample to be assayed in 50 mM Tris-HCl, pH7 (buffer or hirudin standard solution or E. coli extracts) are added to 100 $\mu$l 50 mM Tris-HCl, pH7. Then 5 $\mu$l thrombin (30 IU/ml in 0.85 % NaCl; Behring-Werke) are added. The whole is then carefully mixed. After a 1-minute incubation at room temperature the test tubes are turned by 180°. A positive result, i.e. inhibition of the thrombin, can be seen when the solution flows out of the tube, whereas in the case of a negative result the clotting is not prevented and the fibrin gel remains at the bottom of the tube.

Enriched extract from the E.coli clone No. 9 inhibits the transformation from fibrinogen to fibrin, whereas an equally treated E. coli extract which does not contain the hirudin gene displays no inhibition. A comparison with hirudin standard solutions shows that the biological activity of the hirudin-like protein from E. coli corresponds to a hirudin concentration of 120 ng/ml.

The hirudin activity of the E. coli extracts is moreover measured by their ability to inhibit thrombin activity vis-à-vis a chromogenic substrate. In this reaction thrombin cleaves the yellow compound p-nitroaniline from chromogenic substrate Tos-Gly-Pro-Arg-pNA (Chromozyme TH, Boehringer, Mannheim). In the test 890 $\mu$l diethanolamine buffer (150 $\mu$l/l, pH 8.4) are mixed with 20 $\mu$l of a thrombin solution (5 IU/ml in 0.25 M phosphate buffer, pH 6.7).

To this 50 $\mu$l of a hirudin-containing solution (x ng hirudin in diethanolamine buffer for gauging or E. coli extract) are added and incubated for 5 minutes at 25° C.

Subsequently 125 $\mu$l of a chromozyme solution (1.5 mM in diethanolamine buffer) are added and the extinction of the mixture is measured at 405 nm in the photometer (Shimadzu) every 60 seconds for 6 minutes. The increase of the extinction is a standard for the thrombin activity and is reciprocal to the hirudin activity.

## Claims

1.  The DNA sequence

```
  1  AATTCTATGGTTGTTTACACTGACTGCACCGAATCTGGTCAGAACCTGTGCCTGTGCGAA
     TTAAGATACCAACAAATGTGACTGACGTGGCTTAGACCAGTCTTGGACACGGACACGCTT

 61  GGTTCTAACGTTTGCGGTCAGGGAAACAAATGCATCCTGGGTTCTGACGGTGAAAAGAAC
     CCAAGATTGCAAACGCCAGTCCCTTTGTTTACGTAGGACCCAAGACTGCCACTTTTCTTG

121  CAGTGCGTTACCGGTGAAGGTACTCCGAAAACCGCAGTCTCACAACGACGGTGACTTCGAA
     GTCACGCAATGGCCACTTCCATGAGGCTTTGGCGTCAGAGTGTTGCTGCCACTGAAGCTT

181  GAAATCCCCGGAAGAATACCTGCAATAGTAAGTGAGCGTCGGATC
     CTTTAGGGGCCTTCTTATGGACGTTATCATTCACTCGCAGCCTAG
```

which encodes a hirudin-like protein having the biological activity of hirudin.

2.  Recombinant DNA molecule for cloning comprising a DNA sequence according to claim 1.

3.  Recombinant DNA molecule characterized in that it comprises a DNA sequence according to claim 1 which is operatively linked to an expression control sequence.

4.  Recombinant DNA molecule according to claim 3 characterized in that the expression control sequence is an E. coli promoter system, the E. coli lac system, the E. coli $\beta$-lactamase system, the E. coli trp system, the E. coli lipoprotein promoter, a yeast expression control sequence or another eukaryotic expression control sequence.

5.  Host micro organism characterized in that it is transformed with at least one or the recombinant DNA molecules according to any one of claims 2 to 4.

6.  Host micro organism according to claim 5, characterized in that it is E. coli.

**7.** A process for the preparation of a hirudin-like protein having the amino acid sequence

```
 1  met thr met ile thr asn ser met Val Val Tyr Thr Asp Cys Thr Glu Ser Gly Gln Asn
21  Leu Cys Leu Cys Glu Gly Ser Asn Val Cys Gly Gln Gly Asn Lys Cys Ile Leu Gly Ser
41  Asp Gly Glu Lys Asn Gln Cys Val Thr Gly Glu Gly Thr Pro Lys Pro Gln Ser His Asn
61  Asp Gly Asp Phe Glu Glu Ile Pro Glu Glu Tyr Leu Gln *** ***
```

and the biological activity of hirudin,
characterized in that a host organism is transformed with a recombinant DNA molecule according to claim 3 or 4 and in that the hirudin-like protein obtained by expression and culturing of the transformant is isolated.

**Patentansprüche**

**1.** DNA-Sequenz, die für ein hirudinähnliches Protein mit der biologischen Aktivität von Hirudin codiert.

**2.** Rekombinantes DNA-Molekül für die Klonierung, eine DNA-Sequenz nach Anspruch 1 enthaltend.

**3.** Rekombinantes DNA-Molekül, dadurch gekennzeichnet, daß es eine DNA-Sequenz nach Anspruch 1 umfaßt, die operativ mit einer Expressionskontrollsequenz verknüpft ist.

**4.** Rekombinantes DNA-Molekül nach Anspruch 3, dadurch gekennzeichnet, daß die Expressionskontrollsequenz ein E.coli-Promotorsystem, das E.coli-lac-System, das E.coli-$\beta$-Lactamase-System, das E.coli-trp-System, der E.coli-Lipoprotein-Promotor, eine Hefe-Expressionskontrollsequenz oder eine andere eukaryotische Expressionskontrollsequenz ist.

**5.** Wirtsmikroorganismus, dadurch gekennzeichnet, daß er mit zumindest einem der rekombinanten DNA-Moleküle nach irgendeinem der Ansprüche 2 bis 4 transformiert ist.

**6.** Wirtsmikroorganismus nach Anspruch 5, dadurch gekennzeichnet, daß er E.coli ist.

**7.** Verfahren zur Herstellung eines hirudinähnlichen Proteins mit der Aminosäuresequenz

```
 1   met thr met ile thr asn ser met Val Val Tyr Thr Asp Cys Thr Glu Ser Gly Gln Asn
21   Leu Cys Leu Cys Glu Gly Ser Asn Val Cys Gly Gln Gly Asn Lys Cys Ile Leu Gly Ser
41   Asp Gly Glu Lys Asn Gln Cys Val Thr Gly Glu Gly Thr Pro Lys Pro Gln Ser His Asn
61   Asp Gly Asp Phe Glu Glu Ile Pro Glu Glu Tyr Leu Gln *** ***
```

und der biologischen Aktivität Von Hirudin, dadurch gekennzeichnet, daß ein Wirtsorganismus mit einem rekombinanten DNA-Molekül nach Anspruch 3 oder 4 transformiert wird und daß das durch Expression und Kultivierung des Transformanten erhaltene hirudinähnliche Protein isoliert wird.

**Revendications**

**1.** Séquence d'ADN :

codant pour une protéine analogue à l'hirudine ayant l'activité biologique de l'hirudine.

**2.** Molécule d'ADN recombinant à cloner, comprenant une séquence d'ADN selon la revendication 1.

**3.** Molécule d'ADN recombinant, caractérisée en ce qu'elle comprend une séquence d'ADN selon la revendication 1, fonctionnellement liée à une séquence de régulation d'expression.

**4.** Molécule d'ADN recombinant selon la revendication 3, caractérisée en ce que la séquence de régulation d'expression est un système de promoteur de E. coli, le système lac de E. coli, le système de la β-lactamase de E. coli, le système trp de E. coli, le promoteur de lipoprotéine de E. coli, une séquence de régulation d'expression de levure ou une autre séquence de régulation d'expression eucaryote.

**5.** Micro-organisme-hôte caractérisé en ce qu'il est transformé avec au moins l'une des molécules d'ADN recombinant selon l'une quelconque des revendications 2 à 4.

**6.** Micro-organisme-hôte selon la revendication 5, caractérisé en ce qu'il s'agit de E. coli.

**7.** Procédé de préparation d'une protéine analogue à l'hirudine, ayant la séquence d'aminoacide :

```
 1  met thr met ile thr asn ser met Val Val Tyr Thr Asp Cys Thr Glu Ser Gly Gln Asn
21  Leu Cys Leu Cys Glu Gly Ser Asn Val Cys Gly Gln Gly Asn Lys Cys Ile Leu Gly Ser
41  Asp Gly Glu Lys Asn Gln Cys Val Thr Gly Glu Gly Thr Pro Lys Pro Gln Ser His Asn
61  Asp Gly Asp Phe Glu Glu Ile Pro Glu Glu Tyr Leu Gln *** ***
```

et l'activité biologique de l'hirudine, caractérisé en ce qu'on transforme un organisme-hôte avec une molécule d'ADN recombinant selon la revendication 3 ou 4, et en ce que la protéine analogue à l'hirudine obtenue par expression et culture du transformant, est isolée.

# FIG. 1
## Synthesis Scheme

asnsermetValValTyrThrAspCysThrGluSerGlyGlnAsnLeuCysLeuCysGlu

```
AATTCTATGGTTGTTTACACTGACTGCACCGAATCTGGTCAGAACCTGTGCCTGTGCGAA
TTAAGATACCAACAAATGTGACTGACGTGGCTTAGACCAGTCTTGGACACGGACACGCTT
```

Fragment I

GlySerAsnValCysGlyGlnGlyAsnLysCysIleLeuGlySerAspGlyGluLysAsn

```
GGTTCTAACGTTTGCGGTCAGGGAAACAAATGCATCCTGGGTTCTGACGGTGAAAAGAAC
CCAAGATTGCAAACGCCAGTCCCTTTGTTTACGTAGGACCCAAGACTGCCACTTTTCTTG
```

Fragment II

GlnCysValThrGlyGluGlyThrProLysProGlnSerHisAsnAspGlyAspPheGlu

```
CAGTGCGTTACCGGTGAAGGTACTCCGAAACCGCAGTCTCACAACGACGGTGACTTCGAA
GTCACGCAATGGCCACTTCCATGAGGCTTTGGCGTCAGAGTGTTGCTGCCACTGAAGCTT
```

Fragment III

GluIleProGluGluTyrLeuGln******

```
GAAATCCCGGAAGAATACCTGCAATAGTAAGTGAGCGTCGGATC
CTTTAGGGCCTTCTTATGGACGTTATCATTCACTCGCAGCCTAG
```

Linker

# FIG. 2

Recombinant Plasmid pRudi 1 with Inserted Synthetic EcoRI-BamHI Fragment

metthrmetilethrasnsermetVal          GluTyrLeuGln******

ATGACCATGATTACGAATTCTATGGTT ............ GAATACCTGCAATAGTAAGTGAGCGTCGGATCC
TACTGGTACTAATGCTTAAGATACCAA ............ CTTATGGACGTTATCATTCACTCGCAGCCTAGG

Eco RI          Bam HI
                Sal I
Promotor        Pst I
                Hind III

Origin of
Replication

pRudi I

Intergenic
region from the
bacteriophage L1

alpha
fragment of
the beta
lactamase gene

# FIG. 3

Amino Acid Sequence of the Hirudin Fusion Protein.

1 met thr met ile thr asn ser met Val Val Tyr Thr Asp Cys Thr Glu Ser Gly Gln Asn

21 Leu Cys Leu Cys Glu Gly Ser Asn Val Cys Gly Gln Gly Asn Lys Cys Ile Leu Gly Ser

41 Asp Gly Glu Lys Asn Gln Cys Val Thr Gly Glu Gly Thr Pro Lys Pro Gln Ser His Asn

61 Asp Gly Asp Phe Glu Glu Ile Pro Glu Glu Tyr Leu Gln *** ***